# EUROPEAN PATENT SPECIFICATION

(11) **EP 0 956 334 B1**
(45) Date of publication and mention of the grant of the patent: **26.11.2003**
(21) Application number: 98940648.3
(22) Date of filing: 01.09.1998
(51) Int. Cl.: C12C 11/09, C12M 1/40

(54) **A PROCESS FOR PRODUCING A FERMENTATION PRODUCT**
VERFAHREN ZUR HERSTELLUNG EINES FERMENTATIONSPRODUKTES
PRODUCTION D'UN PRODUIT DE FERMENTATION

(30) Priority: 02.09.1997 JP 25144197
(43) Date of publication of application: 17.11.1999
(73) Proprietor: SAPPORO BREWERIES LTD., Tokyo 150-8686 (JP)
(72) Inventor: UMEMOTO, Shingo Sapporo Breweries Limited, Yaizu-shi Shizuoka-ken 425-0013 (JP); MITANI, Yutaka Sapporo Breweries Limited, Yaizu-shi Shizuoka-ken 425-0013 (JP)
(74) Representative: VOSSIUS & PARTNER
(86) International application number: JP9803892
(87) International publication number: WO99011751

(56) References cited:
- EP-A- 0 508 343
- EP-A- 0 508 344
- EP-A- 0 669 393
- WO-A-86/05202
- US-A- 4 754 698
- US-A- 5 014 612
- S. UMEMOTO ET AL.: "primary fermentation with immobilized yeast in a fluidizer bed reactor" TECHNICAL QUARTERLY MASTER BREWERS' ASSOCIATION OF THE AMERICAS, vol. 35, no. 2, 1998, page 58-61 XP002097635
- A. AIVASIDIS ET AL.: "continuous fermentation of alcohol-free beer with immobilized yeast in fluidized bed reactors" EBC 1991 CONGRESS NOTES MAI 1991 LISBON, 1991, page 569-576 XP002097636

## Description

### Field of Technology

The present invention relates to a process for producing a fermentation product and in particular to a process for producing a fermentation product using a bioreactor wherein a fluidized bed is formed while fermentation being conducted, and a fermentation product is put out and simultaneously a new raw-material solution is fed to the reactor so that the above fermentation is repeatedly carried out.

### Background Technology

Many fermentation processes using bioreactors are directed to an operation method by continuous fermentation (Bioreactor, 112 (1985), Kodansha Scientific). The characteristics of the operation method by continuous fermentation are (1) quality of the product can be easily maintained constant, (2) automatic control is easy, and (3) the operation is suitable for large-scale production. On the other hand, an operation method by batch fermentation is adopted in traditional method for production of alcoholic beverages. The operation by batch fermentation has demerits such as the necessity for the duration for microbial growth (induction phase) in addition to the fermentation time, but is characterized in that complete consumption of fermentative raw materials can be readily achieved as compared with the continuous operation.

Even in processes for producing alcoholic beverages such as beer etc., a large number of processes for producing alcoholic beverages by using bioreactors have been proposed since the technology for immobilization of yeast was developed (Brauwelt, 1491, 111 (1971)).

In these processes, yeast is maintained on an immobilization carrier to raise the density of the microbial cells and is used to reduce the fermentation time significantly. The bioreactors used in fermentation are roughly classified, depending on their types, into continuous stirred tank reactor, packed bed type reactor, membrane type reactor, fluidized-bed type reactor and horizontal reactor (World of Bioreactors, 22 (1992) Hario Laboratory).

It is reported that the type of bioreactor used in fermentation for alcohol formation producing simultaneously a carbon dioxide gas by metabolism of fermentative raw materials, such as primary fermentation of beer, is preferably the fluidized-bed type in order to facilitate discharge of the gas to the outside of the system (Kagaku Kogaku, 543, 60 (1996)).

Application of bioreactors to beer brewing process appears in Finland (Pajunen, E. et al., EBC Proc. 23rd Congr., 361 (1991)), Netherlands (Brauwelt, 133, 302 (1993)) etc. However, in the case where beer was produced by continuous operation using a bioreactor, it was difficult to produce products having flavor similar to those of products produced by the traditional batch fermentation process. Specifically, there was a problem with flavor due to high amino acid and diacetyl levels and low ester levels in beer (New Bioreactor, 116 (1988) CMC, J. Am. Soc. Brew. Chem., 66, 43 (1985)).

Therefore, use of bioreactors is mainly limited in practice to secondary fermentation, and the long-term operation of primary fermentation in practice is still not satisfactorily conducted even in the world.

However, introduction of bioreactors not only into secondary fermentation but also into primary fermentation is essential for practice of efficient beer fermentation, and this has been a problem with application of bioreactors in beer brewing process.

As described above, it has been noted that in beer brewing using bioreactors, there is a problem with flavor of products, and some improved techniques have been proposed therefor. For example, as the measure for reducing amino acid level in beer, there proposed is a continuous fermentation method using a fluidized-bed reactor where fermented wort is circulated to permit amino acids to be effectively consumed by immobilized yeast thereby lowering amino acid level (Japanese Patent Kokai No. 7-123969). According to this method, the consumption of amino acids by the yeast is improved as compared with the packed bed type reactor etc.

In this method, however, it is necessary to limit the amount of wort introduced into the reactor, that is, it is necessary to decrease the degree of dilution of the fermented wort, so the amount of the fermentation product produced per hour is low and high-productivity as a property inherent in the bioreactor cannot be realized.

Alternatively, a method where the metabolism of amino acids and the metabolism of sugars in a fermentative raw-material solution by yeast are continuously conducted in separate reactors is proposed (Japanese Patent Publication No. 6-73445), but this method makes use of a combination of the continuous stirred tank reactor and the packed bed type reactor which are different in reaction mode, and those two types of reactors are further provided therebetween with a centrifugal separator for removal of yeast, thus making the system complicated.

Diacetyl is formed by oxidative decarboxylation reaction from α -acetolactic acid, which is an intermediate in valine biosynthesis, and is a substance which even in a small amount, adversely affects the flavor of beer. This diacetyl is reduced into acetoin of low-threshold value by yeasts during fermentation and maturation. However, the continuous fermentation system using bioreactors differs from the traditional batch fermentation system in respect of the environment in which yeasts are placed, so it is considered that the amount of formed α-acetolactic acid is more than that of the usual biosynthesis and cannot be reduced by yeasts, resulting in increace of an amount of diacetyl remaining in beer.

As the measure for reducing diacetyl levels, a method of inhibiting the formation of α-acetolactic acid by inclusion-immobilizing yeasts into double-layered carriers is reported (J. Ferment. Bioeng., 199, 76 (1993)), but the procedure of immobilizing yeasts is troublesome and there is still the problem to be solved in practice.

Besides, a method of using yeast having an activity of α-acetolactate decarboxylase, produced by gene transfer (J. Inst. Brew., 479, 98 (1992)) is proposed, and this yeast can be used to reduce diacetyl levels, but the disadvantage of said yeast is that the duration of its fermentation ability is shorter than that of its parent strain before gene transfer.

Esters as components important for beer flavor are formed by condensation between acetyl CoA and alcohol by alcohol acetyl transferase which is a cellular membrane enzyme in yeast. This process is an energy-requiring reaction in yeast-and is thus related closely to the growth of yeasts. A fatal disadvantage is noted that the amount of esters formed becomes low, in general, due to a less amount of grown yeasts in a bioreactor system (J. Am. Soc. Brew. Chem., 66, 43 (1985)).

As a measure for increasing ester level, a measure for controlling ester level by fermentation using a fermentative raw-material solution containing an increased content of glucose by enzyme treatment is reported (J. Ferment. Bioeng., 370, 73 (1992)). However, the problem of this method is high costs resulting from use of the enzyme.

For operation of a bioreactor in the continuous fermentation system, besides the above-described problems with flavor and quality, there is a further limiting condition, that is, a raw-material solution should be kept feeding at a predetermined rate to the reactor, and therefore in the case of products from a raw-material solution prepared by the batch system as the pre-step, there are problems of gaps between the steps and of its accompanying adverse effects on the quality of products.

That is, if the bioreactor in the continuous fermentation system is used for the primary fermentation step in beer brewing, sweet wort as the raw-material solution in the bioreactor should usually be retained temporarily because the wort production step as the pre-step is conducted in the batch system. However, it is highly possible that during this retention, the sweet wort may undergo deterioration in quality, particularly due to microbial contamination etc.

To keep the raw-material solution clean without contamination with microorganisms is an essential element for brewing and for long-term operation of bioreactors, so the problem of the gap between the steps is important for adopting the continuous-fermentation-system bioreactor in the primary fermentation step of brewing.

As described above, the reaction system for bioreactors used in producing alcohol and a large amount of carbon dioxide gas etc. is preferably the fluidized bed type. and the carrier used in this fluidized bed type reactor should, as a matter of course, have a high ability to immobilize yeasts, should not inhibit discharge of carbon dioxide gas etc., and should also be excellent in wear resistance and fluidity. In particular, whether the ability of the carrier to discharge carbon dioxide gas etc. is good or not is an important element, and use of carriers poor in this performance causes many practical problems such as reduction in reaction efficiency due to inhibition of contacting the immobilized yeasts to the raw-material solution, inhibition of fluidization of carriers, floating of carriers due to reduction in the apparent density of carriers, and reactor clogging etc. resulting from the floating.

### Disclosure of the Invention

For the above-described reasons, further developments not only in flavor and quality but also in the operating conditions related to the pre-step are desired for application of bioreactors in fields such as beer brewing discharging a large amount of carbon dioxide gas, particularly in application of bioreactors to the primary fermentation step in beer brewing. Further, it is also desirable to establish a fermentation method in which the fermentation time can be reduced and the long-term operation is feasible.

As a result of their eager study to solve the problem, the present inventors developed a process for producing alcoholic beverages such as beer etc. by use of a reactor charged with immobilized yeasts wherein a raw-material solution is circulated to form a fluidized bed while fermentation is conducted, and when or after a fermentation product is put out from the reactor, a new raw-material solution is fed to said reactor so that the above fermentation is repeatedly carried out (the process is referred to hereinafter as the repetitive batch fermentation method). The present inventors found that the fermentation time can be shortened and alcoholic beverages excellent in flavor can be produced by this method. Further, compared to the continuous fermentation system, this method is similar to the batch system operation method as the traditional process for producing alcoholic beverages in respect of the physiological state and growth cycle of yeasts and the distribution of the microbial cells in the reactor etc. The present invention was completed on the basis of such findings.

The first aspect of the present invention is a repetitive batch fermentation process for producing a fermentation product wherein an immobilized microorganism is arranged in a fluidized bed reactor provided with a fluidized bed part, a liquid circulating part and a gas exhaust part, and a raw-material solution is fed to said reactor to conduct fermentation, characterized in that a part of a fermented solution is drawn out from the fluidized bed part and said fermented solution is returned again to the reactor whereby a fluidized bed is formed while fermentation is conducted, and the fermented solution containing the fermentation product is put out from the reactor after fermentation is complete and the fermentation product is put out from the reactor while a new raw-material solution is fed to the reactor so that the above fermentation is repeatedly carried out.

### Brief Description of Drawings

Fig. 1 is a drawing showing one embodiment of the fluidized-bed reactor used in the present invention.
Fig. 2 is a graph showing the change with time in the apparent extract during long-term operation by the repetitive batch fermentation method in Example 1.

### Preferable Embodiments of the Invention

Hereinafter, the present invention is described in detail.

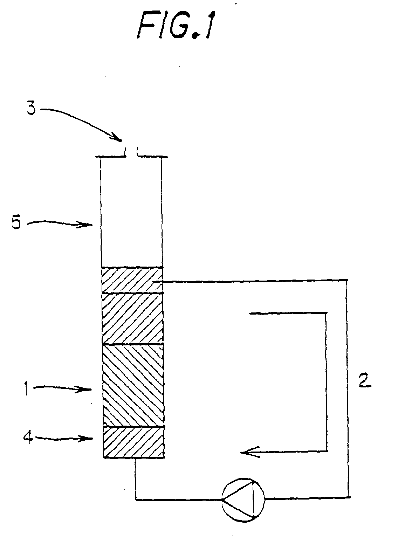

In the present invention, a fluidized-bed reactor is used. One embodiment of this apparatus is shown in Fig. 1. As illustrated therein, the reactor is provided with a fluidized bed part (1), a liquid circulating part (2) and a gas exhaust part (3) and further includes a calming section (4), a hollow cylindrical part (5) etc. The apparatus illustrated therein is suitable for production of carbon dioxide gas-forming fermentation products such as beer etc.

The fluidized bed part is a place where the microorganism-immobilized carrier is arranged, and the liquid circulating part is a place for drawing out a part of a raw-material solution or a fermented solution from the fluidized bed part and then returning it again to the reactor. Usually, the raw-material solution or the fermented solution is returned to the inside of the reactor through the calming section in a lower part of the reactor where the flow of the introduced fluid is regulated. The hollow cylindrical part is a place for separating carbon dioxide gas formed during fermentation and a fermented solution from each other, and specifically it is a region ranging from the liquid level to the gas exhaust part in an upper part of the apparatus. Gases such as carbon dioxide gas etc. separated in the hollow cylindrical part are discharged to the outside via the gas exhaust part in the upper part of the reactor.

A microorganism immobilized in carriers is arranged in the fluidized bed part of the reactor, and a raw-material solution is fed through the calming section to conduct fermentation, but in the present invention, a part of the raw-material solution or fermented solution is circulated whereby a fluidized bed is formed for fermentation. By keeping the immobilized microbial cells at high density in the reactor, fermentation can be finished in a shorter time than fermentation using a non-immobilized microorganism.

The circulation of the liquid for forming the fluidized bed is now described. A part of the fermented solution (or the raw-material solution) is drawn out by a pump through a suitable position of the fluidized bed, for example from the upper part of the fluidized bed such as in the illustrated example and then returned to the reactor through the lower part of the calming section whereby a fluidized bed is formed in the reactor. The position where the fermented solution is drawn out is not limited to the upper part of the fluidized bed, and the manner for drawing out may be in either longitudinal or lateral direction.

The superficial liquid flow rate (linear velocity of fluid per unit volume of the fluidized bed) during fermentation can be varied depending on the density of carriers carrying the microorganism, but usually is 1 to 20 cm/min, preferably 1 to 12 cm/min.

By permitting the fermented solution to be circulated and simultaneously by using carriers hardly retaining gases, the carbon dioxide gas formed during fermentation is not retained in the fluidized bed and is easily discharged from the hollow cylindrical part via the gas exhaust part to the outside of the reactor.

In the process of the present invention, the fermented solution containing the fermentation product after fermentation is put out from the reactor and simultaneously a new raw-material solution is fed to the reactor whereby the fermentation is repeatedly conducted. That is, circulation is stopped after fermentation, and when or just after the fermented solution is put out from the reactor, a new raw-material solution is fed to the reactor whereby the above fermentation is repeatedly conducted.

The method for operating the reactor is the repetitive batch system. The repetitive batch fermentation yields products excellent in flavor in a short time. The speculative reason why product produced by this repetitive batch operation is excellent in flavor as compared with product by the continuous operation is that the growth and renewal of the microorganism is performed during fermentation and that the physiological state and growth cycle of the microorganism and the distribution of the microbial cells in the reactor etc. are similar to those of the batch operation in the traditional process for producing alcoholic beverages.

Various kinds of carries can be used, and especially carriers consisting of chitosan, particularly chitosan beads are preferable. Because chitosan beads are hydrophilic and porous in form, a carbon dioxide gas can be easily discharged therefrom. Further, chitosan beads hardly wear out and have a similar density to that of the raw-material solution, so their fluidity is good. Furthermore, because they can retain a large amount of microorganisms thereon, the fermentation time can be reduced. In addition, the microorganism is adsorbed and immobilized relatively gently thereon, which would facilitate the growth and removal of the microbial cells, and unlike entrapment carriers, inactive microorganisms do not remain in the carriers.

Although sterilization of carriers may be conducted in any arbitrary method, sterilization under a pressure, sterilization with caustic soda, or steam-sterilization is preferable. The method of immobilizing the microorganism onto carriers can also be conducted by adding the carriers to the microbial suspension and stirring or circulating the fluid, but any other methods known in the art can also be used.

The microorganism to be immobilized can be selected depending on the object. For example, if alcoholic beverages such as beer, sparkling alcoholic beverages, sake etc. are to be produced, yeasts such as Saccharomyces cerevisiae, Saccharomyces uvarum etc. can be used.

The raw material for producing fermentation products may be any materials suitable for fermentation by the tested microorganism, and any known materials can be used arbitrarily, but for production of alcoholic beverages, wort, must, priming solution or grain sugar solution is used solely or in suitable combination thereof.

The fermentation products as the object of the present invention include various products, preferably alcoholic beverages, specifically beer, sparkling alcoholic beverages, sake, wine etc., among which beer, sparkling alcoholic beverages and other liquors forming carbon dioxide gas during fermentation are particularly preferable.

### Examples

Hereinafter, the present invention is described in detail with reference to the Examples, which however are not intended to limit the present invention.

### Example 1

As the reactor, a cylindrical column of 2400 mm in height (material: polycarbonate, total volume: 20 L, diameter: 100 mm) was used.

7 L (bulk volume) of chitosan beads (trade name: Chitopearl HP, Fuji Spinning Co., Ltd.) having beer yeast (Saccharomyces cerevisiae) immobilized thereon were put into this reactor and arranged therein. Then, 7 L of a wort adjusted to an extract of 11 % Plato was introduced through the calming section into said reactor, and circulated at 10 °C by setting the superficial liquid flow rate in the reactor at 6 cm/min. thereby forming a fluidized bed to conduct fermentation. The hollow cylindrical part was 6 L.

After fermentation, the fermented wort was put out through an upper part of the fluidized bed, and simultaneously new wort was introduced through the calming section to conduct repetitive batch fermentation. The total operation time was 74 days (38 batches), and the fermentation time per batch was approximately 23 hours, and the amount of the immobilized microorganism was about 2×10⁹ cells/1 ml of carrier.

Free amino nitrogen, total diacetyl, and ethyl acetate in the fermented wort were determined. The results are shown in Table 1. The total diacetyl refers to the sum of the amount of pre-existing diacetyl and the amount of diacetyl forcibly converted from α-acetolactic acid by heating after introducing air to the fermented wort.

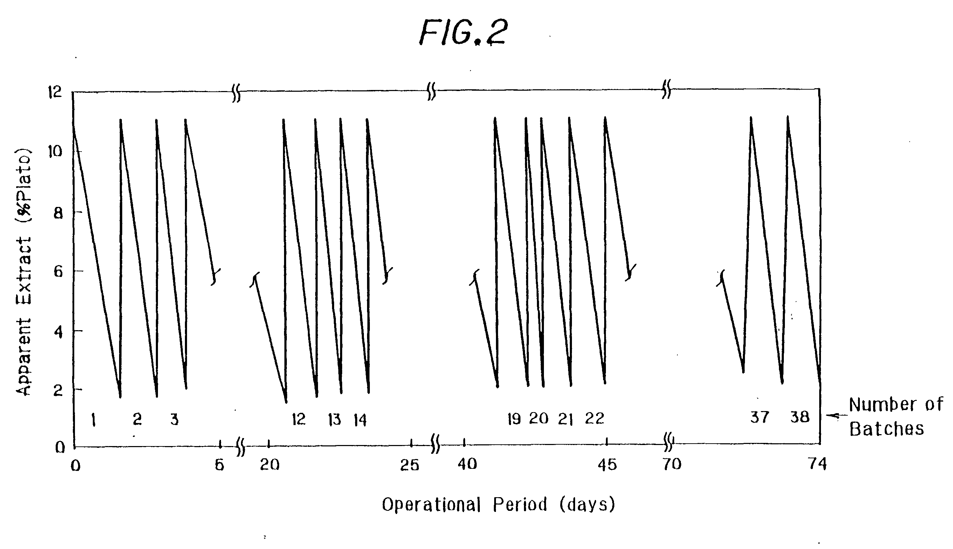

The change with time in the apparent extract during operation for 74 days in total by the repetitive batch fermentation method is shown in Fig. 2.

### Example 2

As the reactor, a cylindrical column of 2400 mm in height (material: polyvinyl chloride, total volume: 80 L, diameter: 200 mm) was used, and 13 L (bulk volume) of chitosan beads (the same as in Example 1) having beer yeast (the same as in Example 1) immobilized thereon were put into this reactor and arranged therein. Then, beer was produced in the same manner as in Example 1 except that the amount of the fed wort was 40 L, the superficial liquid flow rate in the reactor was set at 4 cm/min, the wort was circulated at 8°C, and the hollow cylindrical part was 27 L. The total operation time was 51 days (18 batches), and the fermentation time per batch was approximately 50 hours, and the amount of the immobilized microorganism was about 1 × 10⁹ cells/1 ml of carrier. The results are shown in Table 1.

**Table 1**

| | Example 1 | | Example 2 | |
|---|---|---|---|---|
| Total operation time (days) | 24 | 74 | 41 | 51 |
| Number of batches (batches) | 13 | 38 | 14 | 18 |
| Fermentation time of the batches (hours) | 23 | 24 | 53 | 49 |
| Original extract (%Plato) | 10.95 | 10.69 | 11.37 | 11.23 |
| Apparent extract (%Plato) | 1.80 | 2.05 | 1.97 | 2.28 |
| Free amino nitrogen (mg/L) | 48 | 80 | 82 | 86 |
| Total diacetyl (mg/L) | 0.40 | n. t. | 0.26 | 0.25 |
| Ethyl acetate (mg/L) | 37 | 29 | 26 | 30 |
| n. t. : not tested. | | | | |

As is evident from the table, in both of Examples 1 and 2, free amino nitrogen and total diacetyl are formed in less amounts. When a continuous fermentation method by the continuous plug flow reactor is conducted, the amount of free amino nitrogen was 98 mg/L, the amount of total diacetyl was 0.78 mg/L, and the amount of ethyl acetate was 14 mg/L in the fermented wort.

### Industrial Applicability

According to the present invention, the fermentation time can be reduced and the operation can be conducted for a prolonged period of time in order to produce fermentation products by a fluidized-bed reactor.

In production of alcoholic beverages such as beer and sparkling alcoholic beverages, the repetitive batch fermentation method using a bioreactor is used as the primary fermentation step whereby the operation is made feasible in connection with the wort production step as the pre-step conducted by the batch system, and it is not necessary to retain a sweet wort temporarily and there is no worry about deterioration in quality in the sweet wort. Further, the resulting product has flavoring quality similar to that of products by the conventional batch fermentation.

## Claims

1. A repetitive batch fermentation process for producing a fermentation product wherein an immobilized microorganism is arranged in a fluidized bed reactor provided with a fluidized bed part, a liquid circulating part and a gas exhaust part, and a raw material solution is fed to said reactor to conduct fermentation wherein a part of a fermented solution is drawn out from the fluidized bed part and said fermented solution is returned again to the reactor whereby a fluidized bed is formed while fermentation is conducted, and the fermented solution containing the fermentation product is put out from the reactor after fermentation is complete, and a new raw material solution is fed to said reactor after the fermented solution is put out from the reactor so that the above fermentation is repeatedly carried out.

2. The process according to claim 1 wherein the fluidized bed is formed under the condition of a superficial liquid flow rate of 1 to 20 cm/min.

3. The process according to claim 1 or 2 wherein the immobilized microorganism arranged in the reactor is a microorganism immobilized on a carrier consisting of chitosan.

4. The process according to any one of claims 1 to 3 wherein the immobilized microorganism is yeast.

5. The process according to any one of claims 1 to 4 wherein the fermentation product is an alcoholic beverage.

6. The process according to any one of claims 1 to 5 wherein the fermentation product is beer, sparkling alcoholic beverage, sake, wine or liquors forming carbon dioxide.

## Patentansprüche

1. Verfahren zur Herstellung eines Fermentationsprodukts durch wiederholte Batchfermentation, wobei ein immobilisierter Mikroorganismus in einem Fließbettreaktor angebracht ist, der mit einem Fließbettteil, einem Flüssigkeitskreislaufteil und einem Gasaustrittteil ausgestattet ist, und wobei eine Rohmateriallösung in den Reaktor gefüllt wird, um Fermentation durchzuführen, wobei ein Teil der fermentierten Lösung von dem Fließbettteil abgelassen wird und die fermentierte Lösung wieder in den Reaktor zurückgeführt wird, wobei ein Fließbett gebildet wird, während die Fermentation durchgeführt wird, und die fermentierte Lösung, die das Fermentationsprodukt enthält, nach Beendigung der Fermentation aus dem Reaktor gelassen wird und eine neue Rohmateriallösung in den Reaktor gefüllt wird, nachdem die Fermentationslösung aus dem Reaktor gelassen wurde, sodass die vorgenannte Fermentation wiederholt durchgeführt wird.

2. Verfahren nach Anspruch 1, wobei das Fließbett unter der Bedingung einer Oberflächenflüssigkeits-Flussrate von 1 bis 20 cm/min gebildet wird.

3. Verfahren nach Anspruch 1 oder 2, wobei der in dem Reaktor angebrachte immobilisierte Mikroorganismus ein Mikroorganismus ist, der an einem aus Chitosan bestehenden Träger immobilisiert ist.

4. Verfahren nach einem der Ansprüche 1 bis 3, wobei der immobilisierte Mikroorganismus Hefe ist.

5. Verfahren nach einem der Ansprüche 1 bis 4, wobei das Fermentationsprodukt ein alkoholisches Getränk ist.

6. Verfahren nach einem der Ansprüche 1 bis 5, wobei das Fermentationsprodukt Bier, ein alkoholisches Sprudelgetränk, Sake, Wein oder Kohlendioxid bildende alkoholische Getränke ist/sind.

## Revendications

1. Procédé de fermentation répétitive en batch destiné à produire un produit de fermentation, dans lequel un microorganisme immobilisé est arrangé dans un réacteur à lit fluidisé muni d'un élément à lit fluidisé, un élément de circulation de liquide et un élément d'évacuation de gaz, et une solution de matières premières est fournie au dit réacteur pour conduire la fermentation, une partie d'une solution fermentée étant tirée hors de l'élément à lit fluidisé et ladite solution fermentée étant ré-introduite dans le réacteur par quoi un lit fluidisé est formé tandis que la fermentation se déroule, et la solution fermentée contenant le produit de fermentation est sortie du réacteur lorsque la fermentation est terminée, et une nouvelle solution de matières premières est introduite dans le dit réacteur après que la solution de fermentation a été sortie du réacteur de sorte que la fermentation mentionnée ci-dessus est réalisée de façon répétée.

2. Procédé selon la revendication 1, dans lequel le lit fluidisé est formé dans des conditions de vitesse du flux liquide superficiel comprises entre 1 et 20 cm/min.

3. Procédé selon la revendication 1 ou 2 dans lequel le microorganisme immobilisé arrangé dans le réacteur est un microorganisme immobilisé sur un support constitué de chitosan.

4. Procédé selon l'une quelconque des revendications 1 à 3 dans lequel le microorganisme immobilisé est une levure.

5. Procédé selon l'une quelconque des revendications 1 à 4 dans lequel le produit de fermentation est une boisson alcoolisée.

6. Procédé selon l'une quelconque des revendications 1 à 5 dans lequel le produit de fermentation est de la bière, une boisson alcoolisée pétillante, du sake, du vin ou des liqueurs formant du dioxyde de carbone.
